# EUROPEAN PATENT APPLICATION

(11) **EP 4 144 403 A1**
(43) Date of publication of application: **08.03.2023**
(21) Application number: 21194703.1
(22) Date of filing: 03.09.2021
(51) Int. Cl.: A61N 1/04, A61B 5/24

(54) **CONDUCTIVE LAYER STRUCTURE WITH MULTI-LAYER CONDUCTIVE ARRANGEMENT**

(71) Applicant: SmartMedics Sp. z o.o., 02-566 Warsaw (PL)
(72) Inventor: Wróblewski, Grzegorz, 05-520 Bielawa (PL); Maciejewski, Adrian, 00-384 Warszawa (PL); Koltowski, Lukasz, 03-704 Warszawa (PL)
(74) Representative: Fuchs Patentanwälte Partnerschaft mbB

(57) **Abstract**

Conductive layer structure (10) for application to a surface (12) of a subject, in particular for use in medical products, comprising:
• a first conductive layer (14) having at least one electrically conductive path (16);
• at least a further conductive layer (14) having at least one electrically conductive path (16);
• at least one intermediate layer (24) extending at least partially between the first and further conductive layer (14);
wherein at least portions of the first and further conductive layer (14) are arranged above one another within the layer structure (10);
and wherein the layer structure (10) is elastically stretchable.

## Description

### Field of the invention

The present invention relates to a conductive layer structure for application to a surface of a subject, in particular for use in medical products. The invention also relates to a method for producing the layer structure. The layer structure is configured for obtaining biometric parameters of a subject, in particular for performing electrocardiography (ECG), electroencephalography (EEG), electromyography (EMG), electroculography (EOG), microdefibrillation and/or defibrillation on a subject.

### Background of the invention

In both the medical and non-medical field, a wide range of layer structures comprising electrically conductive elements are employed to interact with a surface of a subject, e.g. a body of a patient.

For example, layer structures in form of electrode patches are used for ECG monitoring (ECG: electrocardiography), i.e. in order to obtain biometric parameters of a subject. Typically, electrode patches comprise electrically conductive (in short: "conductive") paths that connect the electrodes with devices for measuring signals generated at or picked up by said electrodes.

A prior art electrode patch is known from document EP 3 626 158 A1 whose disclosure is incorporated herein by reference in full. This teaching mentions several use cases for prior art electrode patches as well as for the electrode patches disclosed therein. Said use cases may apply to the presently disclosed layer structure as well.

Moreover, layer structures can e.g. be used for EMS-clothing (EMS: electrical muscle stimulation).

Layer structures and in particular electrode patches should fulfill a variety of requirements, such as limited size which translates into a high level of miniaturization and/or compactness.

The compactness relates in particular to a footprint or base area of the layer structure which determines the area that the layer structure occupies at the subject's surface. Limiting said footprint or area may e.g. allow for a less complicated and/or faster attachment of the layer structure at the subject. It may also mean that less surface of the subject is occupied. This leaves room for attaching further layer structures or other medical systems. Also, the limited size may reduce production and material costs.

Yet, it has been observed that the efficiency and reliability of layer structures may suffer when increasing the compactness.

Further it has generally been found that prior art structures can be difficult to apply, e.g. due to being difficult to handle and to position at a patient.

### Object of the invention

It is an object of the present invention to provide a layer structure for application to a surface of a subject that overcomes at least some of the above drawbacks, such as difficult handling, low efficiency and reliability or low compactness.

In particular, it is an object of the present invention to provide a layer structure having a compact design for achieving a sufficiently reliable and/or efficient signal measurement.

Moreover, it is a preferred object of the present invention to provide a layer structure that is easily and quickly attachable to a subject.

These objects are achieved by the subject matters of the independent claims. Optional embodiments and optional features are specified in the dependent claims and in the following description.

### Summary of the invention

An aspect of the invention relates to a layer structure for application to a surface of a subject, in particular for use in medical products, comprising:
- a first conductive layer having at least one electrically conductive path;
- at least a further conductive layer having at least one electrically conductive path;
- at least one intermediate layer extending at least partially between the first and further conductive layer,
wherein at least portions of the first and further conductive layer are arranged above one another or, differently put, are stacked on top of one another with the intermediate layer in between them. In particular, the conductive layers may be arranged so as to overlap and/or cross (but preferably not contact) one another within the layer structure (e.g. when viewed along an axis extending orthogonally to the subject's surface and/or to a plane of the layers). In other words, at least a section of the first conductive layer and a section of the further conductive layer may be arranged congruently within the layer structure.

At least two and preferably all layers, or at least the conductive and/or intermediate layers, may be non-movable relative to one another. Preferably, these layers are directly or indirectly secured to one another e.g. instead of merely lying on top of and being movable relative to one another. Generally, the layer structure may be provided as a single integrated unit whose components are secured within the layer structure and/or to one another. Note that this state may already be provided when not yet attached to a patient and e.g. taking the layer structure out of a packaging.

As a generally preferred feature, the layer structure is elastically stretchable. Yet, the invention also relates to non-elastically stretchable layer structures having the above features and which may be combined with or configured according to any of the further aspects disclosed herein.

Elasticity of the layer structure or the individual layers reduces negative motion artefacts and improves the positioning on the subject. The layer structure can be longitudinally stretchable in a range from 1% to 1000%, preferably from 100% to 900%, more preferably from 200% to 800%, still more preferably from 300% to 500%, still more preferably from 350% to 450%. Each of the layers of the layer structure can be longitudinally stretchable in a range from 1% to 1000%, preferably from 100% to 900%, more preferably from 200% to 800%, still more preferably from 300% to 500%, still more preferably from 350% to 450%. Each of the conductive layers can have an E-modulus from 1.0 to 100.0 N/mm², preferably from 2.0 to 50.0 N/mm², more preferably from 3.0 to 25 N/mm², still more preferably from 5.0 to 15.0 N/mm².

The medical product in which the layer structure can be used can be a medical patch and in particular an electrode patch.

Generally, the inventors have identified a conflict of interest between achieving a compact design and a satisfying efficiency and/or reliability.

For example, for rendering an electrically conductive layer structure more compact, a cross-section of conductive paths within said layer structure may have to be decreased. This allows for densely packaging a plurality of conductive paths.

As a result, however, the applicable voltages and currents are reduced and an ohmic resistance of the conductive paths increases. This reduces efficiency.

Further, the dense packaging may render the conductive paths more prone to noise, e.g. due to crosstalk and/or interferences between adjacent paths. This again limits efficiency and, more precisely, measurement accuracy.

Still further, if a large number of conductive paths are to be integrated in a densely packed layer structure, so far the remaining available space, e.g. for protective layers, is significantly reduced.

Moreover, as a part of achieving a desired reliability, the layer structures should typically have a sufficient defibrillation resistance. This relates to a case where the subject receives electric shocks from a defibrillator and the electrodes of the electrode patch may pick up respectively strong electric signals and/or may generally be electrically exposed to said shocks. In such a scenario, the electrode patch should be configured to e.g. not heat up excessively or to not be damaged otherwise, as this could harm the patient (e.g. by burning his/her skin). For example, breakthrough voltages of e.g. insulative layers of the layer structure should not be exceeded. Also, the electrode patch should as quickly as possible return to a state in which it again precisely measures the subject's electric signals. This may be referred to as defibrillation overload recovery.

Again, defibrillation resistance and overload recovery may be negatively impaired when increasing the compactness of known electrode patch designs.

The presently disclosed solution, on the other hand, helps to solve these conflicting interests as follows: By providing conductive paths in multiple layers, a size and in particular footprint of the layer structure may be reduced, e.g. compared to arranging the paths next to one another in one common layer. This is in particular valid when, according to the above aspect, the paths are stacked above one another, thereby e.g. reducing a width and thus footprint of the layer structure.

Note that a possibly resulting increase in thickness due to increasing the number of layers may be acceptable, e.g. more acceptable than an increased footprint. Differently put, an increasing thickness may not be considered as unduly lowering the compactness of the layer structure, as said compactness preferably concerns the size of the subject's surface area that is occupied by the layer structure. This limited footprint may also be preferable in terms of packaging and/or may reduce an area of possible skin irritations. Also, a smaller footprint on the subject's body leads to smaller stress harvesting during motion which gives lower noise while signals are measured. Smaller footprint also results in smaller skin irritation on the subject's body.

Further, a layer structure that is large in dimensions and in particular in terms of its footprint is difficult to apply and it may require more than one person to do that. A compact one, as presently disclosed, can be easier to apply by one person without additional help or additional adhesive taping. This is of particular relevance when medical personnel acts under stress and time pressure.

Another advantage results from the possibility of providing a limited footprint and a generally reduced size of a connector that is configured to connect to external devices e.g. for signal transmission. Prior art layer structures having only one main layer for accommodating their conductive paths typically connect their often large number of conductive paths next to one another (i.e. in one common plane) to such a connector. Thus, the connector has a large footprint as well which, due to being typically stiffer than the remainder of the patch, will be uncomfortable to wear and difficult to attach to a patient's body. The large connector may also render it difficult or even impossible to place it at a patient's body due to its increased need for free available space.

Alternatively, some prior art solutions use several connectors distributed with the plane of the electrode patch. These are difficult to apply and connect. The present solution preferably only has one connector. Generally smaller connectors compared to the prior art and/or single connectors may be used.

It has also been observed that large single-plane/layer electrode patches can be difficult to produce with existing manufacturing equipment and/or are accompanied with increased manufacturing costs. On the other hand, there is a high chance that the present multi-layer structure can be produced with existing manufacturing equipment due its limited layer sizes (e.g. by stacking conventionally produced and sized layers on top of one another). At the same time, however, functionality and the overall number of conductive paths distributed across the numerous layers is increased.

Further, by distributing the conductive paths across multiple layers, a cross-section of the paths may be increased without increasing a footprint of the layer structure (e.g. compared to respective cross-sections when arranging the paths in one common layer). This way, an ohmic resistance of the paths may be lowered and higher currents and voltages may be applied to the paths. Thus, a high efficiency and accuracy can be achieved while still increasing compactness.

Moreover, by placing the conductive paths in different layers, a distance between them may be increased which e.g. increases defibrillation resistivity. Also, larger volumes of material (e.g. comprised by intermediate layers) may be arranged between them. This may improve electric and/or thermal insulation in both of a horizontal and vertical direction. Further advantageous shielding effects are discussed below.

As a result, the disclosed multilayer structure provides the possibility of making its compact structure still sufficiently resistant for defibrillation impulses. Differently put, the compactness does not negatively decrease defibrillation resistance as a large number of layers and conductive paths can still be present. Also, the generally large number of conductive paths may increase resolution in terms of picking up biosignals, in particular compared to existing single layer structures having similar sizes and footprints.

The increase in distance and/or the provision of dedicated intermediate layers also lowers the risk of local heat concentration. This could theoretically result from (external) defibrillation pulses that are partially picked up by electrodes of the layer structure. Likewise, the improved electric and/or thermal insulation allows for applying high voltages and may help to screen the conductive paths against noise. The latter is in particular valid when, according to a preferred example, dedicated shielding layers are provided that e.g. shield the conductive paths against external electromagnetic disturbances. Note that due to the preferably reduced footprints of the presently disclosed solution, such shielding layers may have a limited size, which reduces costs.

Any shielding layer may be passive (e.g. not electrically driven or not electrically powered) or may be active (e.g. electrically driven or electrically powered). In the latter case, a so called driven guard may e.g. be used.

The layer structure may be an electrode patch and may generally be configured to pick up and/or capture electronic signals and in particular biosignals of a living organism. Thus, the layer structure may e.g. be used for a multiple-lead ECG. For doing so, the layer structure may comprise a number of electrodes. At least some of the conductive paths of the layer structure may each be electrically connected to at least one electrode to carry captured signals e.g. to a connector or to another component of the layer structure.

The electrodes may comprise or be formed by a measurement point according to any of the examples disclosed in EP 3 626 158 A1. Accordingly, the electrodes can e.g. comprise silver or silver-chloride. Further, an electrode may be formed by forming a local electrically conductive connection between a conductive layer (preferably a selected conductive path thereof) and the subject (e.g. by connecting to a surface of the layer structure that is attachable to the subject's surface). This may include providing a channel, recess, aperture or generally a free space between the conductive layer and preferably the selected conductive path thereof in which a conductive substance (e.g. a gel) may be disposed. Note that this may include locally perforating or generally extending through or across any other layers in between a respective conductive layer and the surface that is to be attached to the subject. This may include extending across another conductive layer and/or an intermediate layer arranged in between.

The conductive layers may be configured according to any of the examples disclosed in the above referenced EP 3 626 158 A1. Each conductive layer may comprise a plurality of conductive paths with free spaces between said paths. The paths may cross each other or be locally electrically connected to one another. They may, additionally or alternatively, extend at a distance from and/or alongside one another and may generally be electrically disconnected. Overall, this may result in a net- or grid-like structure of any conductive layer, wherein the paths form respective sections of this structure. Note that even though any conductive layer may thus be discontinuous, it may be considered as defining a defined (discontinuous) layer and/or a defined level of the layer structure. With other words, a plane or level of the layer structure in which conductive paths extend may represent a conductive layer.

On the other hand, the free space within a conductive layer may be filled when providing and preferably laminating an adjacent layer, e.g. a insulative layer. Alternatively, the conductive layer may comprise a (preferably non-conductive) base layer or resin on which the conductive paths are arranged or in which they are embedded.

The conductive paths of the conductive layers can each have a width between 1 mm and 5 mm, preferably between 2 mm and 4 mm. The conductive paths of the conductive layers can each have a width of at least 1 mm, preferably at least 2 mm, still more preferably at least 4 mm. The conductive paths of the conductive layers can each have a width of 5 mm or less, preferably 4 mm or less, more preferably 3 mm or less.

The conductive layers can comprise a conductive particle composition, preferably having an elastic polymer resin, such as a polyurethane, a silicone, a rubber and/or a polydimethylsiloxane resin. In particular, the conductive particle composition can be a silver-carbon paste. Preferably, the silver-carbon paste has at least 50 wt% silver paste so as to ensure a sufficient conductivity. On the other hand, the amount of silver paste of the silver-carbon paste can be reduced, e.g. to less than 90 wt%, preferably less than 80 wt%, so as to reduce costs. On the other hand to maintain the paste's conductivity even under stretching best possibly, the amount of carbon paste can be increased. The silver-carbon paste can have 50 wt% to 90 wt% silver paste, more preferably 60 wt% to 80 wt% silver paste, still more preferably 70 wt% silver paste. The silver paste can have 30 wt% to 80 wt% solid constituents, preferably 40 wt% to 70 wt%, more preferably 50 wt% to 60 wt%. The solid constituents can be the conductive particles of the composition and can have the form of at least one of flakes, powders, particles, micro-particles, nano-particles, nano-tubes, spheres, nano-wires, micro-wires, wires and others. The weight-percentage of the silver-carbon paste that is not silver paste can be carbon paste only or can be carbon paste in addition to other components.

The conductive particle composition can be provided by mixing at least two pastes with different functional phases, such as silver paste and carbon paste, each comprising a polymer resin, wherein the resins have to be compatible. Alternatively, the conductive particle composition can be provided by one paste having at least one functional phase, such as carbon paste only or silver paste only, or by one paste having at least two different functional phases. Alternatively, the conductive particle composition can be provided by one paste having at least one conductive phase, for example conductive polymer paste.

Even though silver and carbon have been mentioned above as exemplary conductive components, principally all conductive materials can be used instead of or in addition to silver and/or carbon, such as Au, Cu and other metals, conductive polymers and/or hydrogels.

To ensure sufficient conductivity, the conductive layers can each have a maximum resistivity of 0.005 Ωm for frequencies from DC to 200 kHz. The resistivity of each of the conductive layers is preferably less than 0.003 Ωm, more preferably less than 0.0005 Ωm, still more preferably less than 0.00001 Ωm, still more preferably less than 0.000001 Ωm for frequencies from DC to 200 kHz.

As the conductive layers can each be elastic, i.e. stretchable, the conductive layers can maintain their conductivity sufficiently even under stretching. For example, a conductive path having a width of 4 mm, when longitudinally elongated to a length of 150% of its original length, can still have a resistivity of less than 0.5 Ωm, preferably less than 0.1 Ωm, more preferably less than 0.05 Ωm, still more preferably less than 0.03 Ωm, in particular while when longitudinally elongated to a length of 175% of its original length, can still have a resistivity of less than 1.0 Ωm, preferably less than 0.75 Ωm, more preferably less than 0.5 Ωm, still more preferably less than 0.3 Ωm, and in particular while when longitudinally elongated to a length of 200% of its original length, can still have a resistivity of less than 10 Ωm, preferably less than 5 Ωm, more preferably less than 2.5 Ωm, still more preferably less than 1.7 Ωm.

Further, a conductive path having a width of 2 mm, when longitudinally elongated to a length of 150% of its original length, can still have a resistivity of less than 0.5 Ωm, preferably less than 0.25 Ωm, more preferably less than 0.1 Ωm, still more preferably less than 0.05 Ωm, in particular while when longitudinally elongated to a length of 175% of its original length, can still have a resistivity of less than 1.0 Ωm, preferably less than 0.75 Ωm, more preferably less than 0.5 Ωm, still more preferably less than 0.3 Ωm, and in particular while when longitudinally elongated to a length of 200% of its original length, can still have a resistivity of less than 15 Ωm, preferably less than 10 Ωm, more preferably less than 8 Ωm, still more preferably less than 7.5 Ωm, still more preferably less than 5 Ωm.

Further, a conductive path having a width of 4 mm, when longitudinally elongated to a length of 150% of its original length, preferably does not increase its resistivity more than 10000%, more preferably not more than 7000%, still more preferably not more than 5000%, still more preferably not more than 4000%.

Further, the conductive path having a width of 2 mm, when longitudinally elongated to a length of 150% of its original length, preferably does not increase its resistivity more than 12000%, more preferably not more than 10000%, still more preferably not more than 8000%, still more preferably not more than 6000%.

Referring to the silver-carbon paste, it has been observed that a silver-carbon paste with a higher amount of carbon is more resistant for stretching in view of its conductivity, i.e. such a paste has a lower increase of resistivity when stretched.

Generally, any layer of the layer structure may be homogeneous, for example, in terms of structure, material or texture. Preferably, said layer may comprise any of the materials disclosed herein, but not combinations thereof. This may support a reliable and predictable deformation.

Likewise, any layer of the layer structure can be continuous, such as a closed film, coating, etc., or can be non-continuous, such as a discontinuous structure, pattern, film, coating, etc. that is only provided in some areas of a plane. Hence, it is apparent that the layers of the layer structure according to the invention, which layers are described above and hereinafter, are not necessarily present in all regions of the layer structure. For example, some of the layers can only be provided in the region of the layer structure where an electrode is formed. Yet, there can be regions of the layer structure, where all of the defined layers are provided.

The number of conductive layers and/or the number of overall layers of the layer structure may e.g. range between 2 and 300, preferably between 2 and 100 or 2 and 50. The number of conductive paths within each layer may range e.g. between 1 and 300, preferably between 1 and 100 or 1 and 50.

Any layer of the multi-layer structure and in particular the conductive layer may have a thickness of e.g between 0.5 µm and 2000 µm, preferably between 1 µm and 1000 µm or between 10 µm and 100 µm.

A conductive path width may e.g. range between 0.1 mm to 10 mm, preferably between 0.5 and 5 mm.

The distance between conductive paths that are arranged in a common layer may e.g. range from 0.1 mm to 20 mm, preferably between 0.5 mm and 15 mm. It has been found that an undesired exceeding of breakdown voltages can reliably be avoided at distances of more than 0.5 mm, e.g. more than 1 mm or at least 2 mm. On the other hand, the more intermediate layers are present and/or the thicker or more insulative these layers are, the closer may the conductive paths be positioned to one another. Further, in order to achieve a compact design, the distance between conductive paths can be less than 20 mm, preferably less than 15 mm, still more preferably let than 10 mm.

Moreover, a generally preferred radiolucency of any conductive layer can be adjustable by varying or selecting the conductive particle composition. Regarding the exemplary silver-carbon paste, a higher amount of carbon paste leads to a higher radiolucency of the silver-carbon paste. However, even a conductive layer consisting completely of silver paste can be sufficiently radiolucent when, e.g., polymer resin comprising conductive silver particles, wherein the conductive layer is relatively thin. For example, thin can describe a thickness of at least less than 100 um, preferably less than 5 um, more preferably less than 25 um. Such a thin conductive layer can be realized by providing a respective composite deposition.

Additionally or alternatively, the layer structure may comprise any of a carrier layer (e.g. providing a stiffening and/or stabilizing effect and preferably forming an outer layer of the layer structure that e.g. faces away from the subject) and a description layer (e.g. visualizing at least one of a plurality of measurement points and/or at least one instruction and/or at least one reference point for supporting the predetermined positioning of the layer structure on the subject).

There may be a plurality of intermediate layers. There may be an adhesive layer that forms or is exposable at an outer surface of the layer structure. The adhesive layer may be configured to adhere to the subject's surface to secure the layer structure thereat.

Generally, the layer structure and/or any layer comprised thereby can be transparent and/or radiolucent.. At least 90% of the area of the layer structure may be radiolucent e.g. by means of radiolucency of an optional adhesive layer and/or radiolucency of any of the conductive layers. Preferably, at least 95% of the area of the layer is radiolucent, more preferably at least 97%. Still more preferably 100% of the area, i.e. the complete area, of the layer structure is radiolucent. As the layer structure preferably has a substantially flat shape or configuration, the area of the layer structure refers to a surface area of the layer structure.

The term radiolucent as used herein refers to materials or composite materials, which are substantially or fully transparent to electromagnetic, magnetic and/or electric field and/or radiation employed in common (medical) imaging procedures, such as X-ray or MRI, in a manner and degree similar to human soft tissue, e.g. muscle tissue. In other words, the layer structure according to the invention does not block electromagnetic radiation of X-ray or MRI but allows it to pass, thereby not interferingly emerging in X-ray or MRI pictures. Thus, the layer structure according to the invention can be worn by a patient during X-ray and/or MRI treatment without negatively affecting the treatment. In particular, radiolucency can mean that the layer structure, i.e. the radiolucent material, does not darken an image of a common hospital X-ray (RTG) and/or of fluoroscopy and X-ray films taken during angiographies and/or other cardio/neuro/radiological procedures (diagnostic and/or therapeutic) more than 60% of image intensity. An exemplary system for common hospital X-ray can be Siemens Artis Zee, with exemplary settings of the lamp voltage from 40 kV to 70kV and lamp current from 8 mA to 12 mA. This maximum darkening should not be exceeded so that the practitioner is still able to assess and evaluate the image. In particular, the radiolucency can mean that the layer structure, i.e. the radiolucent material, does not darken an image of a common hospital X-ray (RTG) more than 50% of image intensity, preferably not more than 40%, more preferably not more than 30%, still more preferably not more than 28%, still more preferably not more than 18%, still more preferably not more than 5%. The less the image intensity is darkened by the layer structure, the better the obtained image can be assessed and evaluated by the practitioner. In other words, radiolucency preferably means that the layer structure, i.e. the radiolucent material, does not attenuate the radiation more than 40.0 µGy/min, preferably not more than 30.0 µGy/min, more preferably not more than 26.0 µGy/min, still more preferably not more than 23.0 µGy/min. In other words, radiolucency preferably means that during a procedure the ratio of dose of radiation attenuated by the layer structure, i.e. the radiolucent material, to overall dose of radiation radiated does not exceed 5%, preferably does not exceed 3.5 %, more preferably does not exceed 2%, still more preferably does not exceed 1.7%, still more preferably does not exceed 1.5%.

It will be understood that the exact radiolucency may be different in different regions of the layer structure, depending, e.g., on the number, thickness and type of layers present in the particular region. For example, the adhesive layer's radiolucency can be greater than the conductive layer's radiolucency.

In an embodiment, the layer structure can further comprise the above-mentioned description layer visualizing at least one of a possible plurality of electrodes and/or at least one instruction and/or at least one reference point for supporting the predetermined positioning of the layer structure on the subject. The description layer can be disposed on a carrier layer, i.e. between a carrier layer and a conductive layer. Preferably, the description layer or the description and a conductive layer is/are the only layer/layers in the layer structure that are not transparent. Consequently, when applying the layer structure to the subject, the user is able to see the description layer through the optional carrier layer and can see the subject through the carrier layer and e.g. an adhesive layer (and preferably any conductive layer) and can thus match portions of the description layer with predetermined marks, reference points, anatomic features, etc. For example, intercostal and sternal lines can be used as reference points, thus preventing the common mistake of placing V1/V2 electrodes at the second intercostal space instead of at the fourth intercostal space. Thus, the positioning is simplified and the layer structure can be positioned more precisely and more quickly. The improved positioning also enhances the reproducibility and consistency of measurements conducted with the layer structure.

Preferably, at least 80% of the layer structure (referring to its surface area) can be at least 15% to 90% optically transparent, preferably at least 40% optically transparent, i.e. can have light transmission from 15% to 90%, preferably at least 40%, for visible light with a wave length between 370 nm to 700 nm.

Further, the layer structure and in particular any layer combination thereof not including the conductive layer can have light transmission from 15% to 90% for visible light with a wave length between 370 nm to 700 nm, more preferably from 30% to 70%, still more preferably from 40% to 55%. More preferably, the light transmission of such a layer combination for light with a wave length of 680 nm is greater than the light transmission for light with a wave length of 550 nm. Wherein in particular, the light transmission of such a layer combination for light with a wave length of 680 nm is 1% to 15% greater than the light transmission for light with a wave length of 550 nm (i.e. percentage light transmission for 680 nm = percentage light transmission for 550 nm plus 1% to 15%), preferably 3% to 10% greater, more preferably 5 to 8% greater. Preferably, for light with a wave length of 680 nm the light transmission of such a layer combination is from 20% to 90%, still more preferably from 30% to 80%, still more preferably from 40% to 60%, still more preferably from 45% to 55%.The intermediate layer can have a refractive index in the range of 0.7 to 2.5, preferably 1.0 to 1.8, more preferably in the range of 1.2 to 1.6, still more preferably about 1.4.

The stretchable character of the layer structure supports a quick and precise attachment of the layer structure at the subject's surface. For example, the layer structure may be stretched (e.g. while already being partially attached to the subject's surface) to better adjust to the surface's shape and/or precisely cover a desired region of the surface. Thus, the layer structure is usable for different subject sizes and shapes.

To support a stretching of the layer structure, preferably each layer thereof including the conductive layers is stretchable as well. Note that an elastic stretching is generally preferred, i.e. a reversible elastic deformation. This is different from providing a non-rigid flexible layer structure that e.g. deforms under its own weight, but that is not stretchable, let alone elastically stretchable. This e.g. applies to typical flexible circuit boards. Generally, the disclosed layer structure may be non-rigid and flexible as well. It may e.g. comprise an at least somewhat elastically stretchable flexible circuit board.

A generally preferred elasticity of the layer structure or its individual layers reduces negative motion artefacts and improves the positioning on the subject. The optional elastic adhesive layer can be longitudinally stretchable in a range from 1% to 1000%, preferably from 100% to 900%, more preferably from 200% to 800%, still more preferably from 300% to 500%, still more preferably from 350% to 450%. Any elastic conductive layer can be longitudinally stretchable in a range from 1% to 1000%, preferably from 100% to 900%, more preferably from 200% to 800%, still more preferably from 300% to 500%, still more preferably from 350% to 450%. The elasticity of both the adhesive layer and the conductive layer allow for an optimal application and adaption to the subject's shape and dimensions, for example to a patient's body. Thus, the layer structure is usable for different subject sizes and shapes.

In an embodiment, the optional adhesive layer can have an E-modulus from 1.0 to 600.0 N/mm2, preferably from 2.0 to 500.0 N/mm2, more preferably from 3.0 to 250.0 N/mm2, still more preferably from 3.0 to 20.0 N/mm2. Any conductive layer can have an E-modulus from 1.0 to 100.0 N/mm2, preferably from 2.0 to 50.0 N/mm2, more preferably from 3.0 to 25 N/mm2, still more preferably from 5.0 to 15.0 N/mm2. The E-modulus of the adhesive layer can be greater than the E-modulus of any conductive layer, in particular the ratio between the adhesive layer's E-modulus and any conductive layer's E-modulus can be in the range from 0.6 to 10.0, preferably in the range from 0.6 to 6.0 or 1.0 to 6.0, more preferably in the range from 0.6 to 1.3, still more preferably in the range from 1.0 to 1.3..

A reliable elastic stretching may be achieved when at least one layer of the layer structure (e.g. the intermediate layer) comprises a deformable polymer. For example, said layer may be configured as or comprise a polymer layer, such as a thermoplastic polymer layer, in particular at least one of a TPU layer, a PET layer, a silicone layer, etc. Alternatively or additionally, any layer of the layer structure may comprise other materials such as papers or textiles. The layer can be non-woven, woven, a film, a foam, a coating, etc. Also, the polymer layer can be siliconised. This can for example be achieved by casting the polymer resin onto a siliconised paper during manufacturing. Such paper can also determine the roughness of the layer. Exemplary roughness (tested according to ISO 4287:1999) of a resulting layer may be in the range from 1 to 1.5 µm Pa, 1.2 to 1.7 µm Pq, in the range from 6.4 to 6.9 µm Pz and 3.6 to 4.1 Pp.

The intermediate layer may comprise material(s) that is (are) different from materials of the conductive layers. As detailed below, the intermediate layer may have different electric characteristics compared to the conductive layers and may e.g. have a lower conductivity or no conductivity at all (i.e. it may be insulative). The intermediate layer may be attached to at least one of the conductive layers by lamination or by printing or by generally depositing the conductive layer (in particular the conductive paths that compose said layer) onto the intermediate layer.

The intermediate layer may e.g. be provided by inserting it as an additional layer that is e.g. made from paper, impregnated paper, plastic film (e.g. polypropylene film) or a plastic plate. Alternatively, it may be provided as a special coating (e.g. parylene) deposited (e.g. printed, casted, spin coated, vacuum deposited) directly on a conductive layer. Following that, a second conductive layer may be provided (e.g. printed) on top of the intermediate layer. Further examples of methods for producing the layer structure are given below.

An adhesive comprised by an optional adhesive layer may be provided as an adhesive film that is e.g. deposited on a support layer comprised by the adhesive layer. The adhesive can be elastic (stretchable). The adhesive can be an inherent property of the adhesive layer. The adhesive can be provided locally in certain areas of the adhesive layer or can cover substantially the whole surface of the adhesive layer. The adhesive can be printed, dispensed, sprayed or deposited by means of other methods. The adhesive can be skin friendly so as to avoid skin irritation and enhance the comfortability of the layer structure. In particular, the adhesive can be biocompatible. Preferably, the ratio of cell survival of cells in contact with the adhesive during cytotoxicity test can be greater than 0.7, preferably greater than 0.8, more preferably greater than 0.9, tested in line with DIN EN ISO 10933-10:2010.

As previously stated, the presently improved defibrillation resistance of the layer structure may comprise or be determined as a defibrillation overload recovery ability. This ability may e.g. require that, after having applied a defibrillation pulse to the subject (and e.g. after the passing of a defined time interval), electrodes of the layer structure that are preferably not connected to another voltage source should not carry a residual potential above a defined threshold. For example, a pair of electrodes that is connected to the subject and/or at least indirectly to one another (e.g. by a conductive gel applied to the subject's surface, i.e. connected gel-to-gel), may have a maximum absolute polarisation potential of 100 mV after a defibrillation attempt. The potential difference may be measured a couple of seconds after the defibrillation attempt, e.g. after five seconds. This way, it is ensured that signals measured by the electrodes actually stem from the subject and are not dominated by residual potential differences resulting from the prior defibrillation overload.

Presently, any electrodes that are e.g. connected gel-to-gel may provide the desired defibrillation overload recovery ability and/or may be examined to verify that said defibrillation overload recovery ability is present.

Additionally or alternatively, defibrillation resistance may include and/or be determined as the ability to maintain a stable residual polarization potential between electrodes of the layer structure after a defibrillation attempt. This may e.g. include that a rate of change of said potential does not exceed a defined threshold (for example during a defined time span following a defibrillation attempt). For example, the rate of change may be limited to +/- 1 mV/sec within a time interval of several seconds, e.g. 30 seconds after measuring the above polarization potential. This e.g. allows for a precise automatic resetting of an overall ECG system after a defibrillation attempt. Generally, it may help to limit undesired effects occurring in the ECG system, such as baseline drift or baseline deviation from a pre-defibrillation state above an acceptable threshold (e.g. less than 0.5 mV).

Additionally or alternatively, defibrillation resistance may include and/or be determined as the above electrode pair having a maximum impedance, e.g. of 10 KΩ, preferably 5KΩ and more preferably 3 kΩ after a defibrillation pulse. This may be measured at a 10 Hz setting of an ECG system.

A possible test set-up for determining whether the layer structure fulfils any or each of the above requirements may include exposing the layer structure (e.g. including at least one electrode pair of the above kind and e.g. attached to a suitable test surface) to at least one discharging capacitor or a number of consecutively discharging capacitors. In a preferred example, at least four consecutively discharging capacitors are provided that may e.g. discharge consecutively after time intervals of up to two minutes, preferably up to one minute and more preferably up to 30 seconds (e.g. between 15 and 30 seconds). Any of the above characteristics (potential difference, rate of change thereof, impedance) may be determined after each discharging of one of the capacitors. Suitable capacitors are, for example, 10-µF capacitors that are charged to 200 V and are discharged through the electrode pair preferably with an ohmic resistance of 100 Ω in series.

According to a preferred embodiment, the intermediate layer is an insulative layer or, in other words, is non-conductive. This may help to provide a desired electric insulation between the conductive layers. If coated with another conductive that is creating a shield/guard, it may also provide a shielding effect, e.g. against undesired influences of electromagnetic radiation. Further, the intermediate layer may act as a spacer and/or a thermal insulator, thereby e.g. improving a defibrillation resistance of the layer structure.

Generally, an electric insulation between each layer and preferably between at least one pair of adjacent conductive layers can amount to at least 10 GO insulation resistance, preferably at least 15 GΩ, more preferably at least 18 GΩ, still more preferably at least 20 GΩ, still more preferably at least 50 GΩ, still more preferably at least 100 GΩ. This insulation may be provided by the at least one intermediate layer.

Additionally or alternatively, a breakdown voltage of the intermediate layer can be at least 100 V, preferably at least 500 V, more preferably at least 1 kV, still more preferably at least 2 kV, still more preferably at least 2.5 kV, still more preferably at least 4 kV, still more preferably at least 6 kV.

According to a preferred example, the layer structure comprises at least one layer that is configured to provide a shielding effect for at least one of the first and further conductive layer, the shielding effect relating to limiting (or preferably fully suppressing) at least one of the following:
- a forming of electrically conductive connections, e.g. between at least one conductive path of a conductive layer and structures outside of said layer;
- an electrostatic coupling e.g. between at least one conductive path of a conductive layer and structures outside of said layer;
- an electromagnetic induction, e.g. between at least one conductive path of a conductive layer and structures outside of said layer, wherein the conductive path may e.g. act as a secondary winding or secondary structure that could (without the shielding effect) be excited by means of an external primary winding or primary structure;
- a radio frequency interference that could e.g. be picked up by a conductive path of a conductive layer and add noise to electric signals carried thereby;
- environmental impacts, such as humidity, temperature, dust or gases.

For achieving any of the above shielding effects, the layer may have appropriately selected material characteristics. In case of the first four shielding effects above, these characteristics may be or relate to electric properties of the layer, such as its ohmic resistance or electromagnetic permeability. For the shielding against environmental impacts, the layer may have a low thermal conductivity and/or be water-, gas- or dust-tight. For example, with respect to water tightness, the layer may fulfill any IP (Ingress Protection) class from 1 to 8 and with respect to dust tightness any IP class from 5 to 6 and/or especially provide tightness against dust sizes from 1µm to 100 µm.

In one example, the layer may have a screening factor against radio waves from 1 to 40 dB (about 1.26 to 10000 times), more preferably from 5 to 35 dB (about 3.16 to about 3162 times), still more preferably from 10 to 30 dB (10 to 1000 times). Generally, for achieving a shielding against electromagnetic radiation, the layer preferably has a low ohmic resistance, that may e.g. be similar to that of the conductive layers (for example not deviate therefrom by more than 20% or more than 10%).

The layer may be one of the conductive layers or the intermediate layer. The intermediate layer may e.g. provide the non-conductive shielding effect and/or a thermal insulation. The conductive layers, on the other hand, may at least partially shield one another against electromagnetic induction, e.g. due to having low ohmic resistances.

Accordingly, in a preferred example, the layer providing the shielding effect is formed by one of the first and further conductive layer and provides a shielding effect for the respective other of the first and further conductive layer. Additionally or alternatively, shielding may be provided by any conductive layer or conductive section comprised by the layer structure, e.g. also layers or sections that are not provided for carrying biosignals. In one example, such shielding layers or sections may be at least partially extend vertically to the layer structure, e.g. to extend along or surround a conductive layer that is to be shielded in a vertical direction.

Additionally or alternatively, there may be another layer and in particular a dedicated shielding layer that provides any of the above shielding effects. Said layer may be arranged between the conductive layers or between any of the outermost conductive layers and a respectively adjacent external surface layer of the layer structure. In one example, the shielding layer is comprised by or forms a respective external surface layer. By providing a dedicated shielding layer, the desired shielding effects may be achieved particularly reliable.

According to a preferred example, at least one of the first and further conductive layer is free of active electric components or comprises more passive electric components than active electric components. In line with established definitions, a passive electric component may be a component that provides its desired functions without the need for an external power supply (e.g. may be a resistor, transformer or conductive path). An active electric component, on the other hand, may require an external power supply and may e.g. be a transistor or a microcontroller. An active electrical component may use supplied electric power to control or modify electric signals.

Using more or exclusively passive electrical components may improve elastic stretchability as active electric components are typically less or even undeformable. Further, this may lower the production costs of the layer structure (e.g. due to not including comparatively expensive microcontrollers). Thus, the layer structure may be economically usable as a disposable element.

An optional aspect includes that the first and further conductive layer are conductively connected to one another. For example, at least one conductive path of the first conductive layer may be connected to a conductive path in the further layer. This may be done by forming a dedicated conductive connection in or across the intermediate layer, e.g. in order to connect several measuring points to one channel and/or to provide an antenna. Other than that, the intermediate layer may be non-conductive. Apart from such optional local connections, it may electrically isolate the conductive layers and their conductive paths from one another. Alternatively, there may be no respective connections and the conductive layers may be fully isolated from one another.

Additionally or alternatively, the first and further conductive layer may be conductively connected or connectable to a common component, such as to a connector that is conductively connected or connectable to an internal or external device. This connection may allow for transferring signals from the conductive paths to the external device via the connector. This way, costly electrical components and in particular active components (in particular logic components or microcontrollers need for signal analysis) may be provided externally from the layer structure. Again, this may lower the costs of the layer structure and render it economically disposable.

The connector can be a polymer based connector that is radiolucent. Thus, it doesn't require removal of electrode for X-ray, MRI or CT examination. Further, the connector can be optically transparent. The connector can be a single socket connector. In some preferred embodiments, the connector can be configured to bare the conductive paths in a direction facing away from the subject in an attached state of the layer structure. This applies in particular for an embodiment in which the layer structure comprises a carrier layer that covers any of the conductive layers towards the layer structure's surface facing away from the subject in an attached state of the layer structure. The connector can be a polymer plate having conductive traces on its surface that faces away from the subject in an attached state of the layer structure. The connector is mounted to the layer structure so that its conductive traces face and partially overlap the conductive paths of the conductive layer, more precisely one conductive trace of the connector partially overlaps only one corresponding conductive path of the conductive layer, thereby reversing the facing direction of the conductive paths from towards the subject to a direction away from the subject in an attached state of the layer structure. The conductive traces of the connector can have the same composition as the conductive layer.

By realizing all of the layer structure's conductive connections via the conductive layers, the optional substance (e.g. hydrogel) of the electrodes and the conductive traces of the connector, the layer structure can be entirely cable-free, which increases the comfort for the subject the layer structure is applied to. Further, it ensures that electrodes do not fall off inadvertently. Moreover, less cables lead to less electromagnetic interference harvesting.

In a further embodiment, the device is magnetically and/or mechanically, removably connectable to the connector. The connector can be provided with one or more magnets, a snap connector, an adhesive, or a clip connector, while the device can be provided with the corresponding counterpart so as to establish a removable connection. Alternatively, the device and the connector can be a single integrated component. Preferably, the device comprises a transmitter so as to wirelessly transfer the signals obtained by means of the layer structure to a processor or analysis unit. For the wireless transmission radio communication like bluetooth, near field communication, WLAN, ZigBee, Z-Wave, LoRa and/or GPRS can be used. Alternatively, the device can transmit the signals via cables. The device can be used for an automated method of interpretation of the signals. Also, continuous monitoring can be carried out with live wireless transmission of the obtained signals from the layer structure to a remote device, such as a smartphone, tablet, laptop, etc.

The layer structure may be deformable and may in particular be bendable. This may help to orient the layer structure as needed for connecting it e.g. to some connector.

For example, according to a further aspect that is not limited to the multi-layer design disclosed herein but may also be used in differently configured layer structures, the conductive layers may have dedicated ends or connecting sections (e.g. conductive traces or ends as explained above and/or connecting electrodes) for connecting to a connector as generally described above. These connecting sections may be oriented closer to a patient's skin when applying the layer structure than e.g. to a back-surface, an outer surface or a substrate of the layer structure that faces away from the skin. Specifically, the connecting sections may be provided close to or at or within an underside of the layer structure that e.g. initially faces the patient's skin when attaching the layer structure thereto. In this state, the connecting sections may not be accessible from above (i.e. from an upper side of the layer structure) for connecting a connector thereto, e.g. because of a number of intermediate layers extending in between the upper side and said connecting sections.

Nonetheless, to connect the connector, the layer structure may generally be configured to be bent, e.g. at least sectionally, so that at least a portion of the layer structure comprising the connecting sections may be flipped and/or re-oriented so as to be arranged at an increased distance to the patient's skin. This portion may not be adhered to the patient's skin but may be bent and moved relative thereto.

For example, an edge portion of the layer structure comprising said connecting sections may (e.g. after initially extending along or in parallel to and/or after preferably facing the skin when applying the layer structure) be bend of folded backwards and/or away from the skin.

The portion comprising the connecting sections to which the connector is to be attached may even be bent or folded to such a degree that it defines the portion of the layer structure having the largest distance to the patient's skin. For example, this portion may be bent by 90° or more relative to an extension in parallel to the skin, e.g. so as to define a C-shape (e.g. a C-shaped cross-section of at least a portion of the layer structure). The bending may also be referred to as flipping the connecting sections and/or the portion of the layer structure to which the connector is to be attached due to part of the layer structure's underside thus becoming part of an upper side of the respectively bent layer structure.

Summarizing the above, a further aspect of this disclosure concerns a conductive layer structure for application to a surface of a subject, in particular for use in medical products, comprising:
- at least one conductive layer having at least one electrically conductive path, said conductive path comprising or being connected to at least one connecting section for conductively connecting the layer structure to a connector (e.g. a connector of any of the kinds disclosed herein);
wherein at least a section of the layer structure (e.g. the section comprising the connecting section) is foldable so that an orientation of the connecting section may be adjusted. This adjustment may include any of the above features and may e.g. result in the above-described at least sectional flipping of the layer structure, in particular of an edge section thereof.

Also, a further aspect of this disclosure concerns a method of applying a conductive layer structure according to the above aspect or any of the aspects concerning a multi-layer design as disclosed herein, the method comprising: applying the layer structure to a patient's skin, folding a potion of the layer structure comprising a connection section for connecting the layer structure to a connector (e.g. a connector of any of the kinds disclosed herein) and connecting the connector to said folded portion. Said folding (or bending) may be implemented according to any of the manners described herein.

The conductive layer structure and the method according to the above further aspects may be combined with and/or further comprise any of the features disclosed herein in connection with a multi-layer design.

According to a preferred embodiment, at least one of the layers of the layer structure comprises or is attached to (e.g. laminated to) a thermoplastic polymer material, in particular a thermoplastic polyurethane material. This may in particular be a layer to which a conductive layer is adhered to (or is formed thereon) or a base layer of the conductive layer on which the conductive paths are provided (e.g. printed or deposited). By using a respective material, the preferred elastic stretching can be achieved.

Preferably, at least one of the first and further conductive layer has a conductive path that is electrically connected to and/or is part of an electrode for measuring electric signals at (or of) the surface. Differently put, at least one of the conductive layers preferably comprises or is connected to an electrode that is configured to capture electric signals of the subject. The electrode may e.g. be formed by a measurement point of a conductive layer (e.g. formed by an end or an exposed section of a conductive path) and by a recess that extends from said point to an external surface of the layer structure (i.e. that forms a connecting channel or free space between said surface and the measurement point). Also, a conductive substance, such as a hydrogel, may be provided in the recess and preferably form part of the electrode as well.

In sum, a recess may be provided which extends from the measurement point across all layers between the measurement point and the subject's surface.

Further, any section of layer that is perforated by the recess and/or borders on the recess (e.g. forms a wall portion thereof) may be considered as a part of the electrode.

Note that due to the conductive layers extending above one another, this may also mean that a recess extends from an upper conductive layer through a lower one towards the subject's surface (the terms upper and lower referring to distances from the surface).

The recess may provide access to only one measurement point or may be sufficiently wide to provide access to a plurality of measurements points (e.g. by spanning across a respective plurality of measurement points).

Additionally or alternatively, in order to provide access to certain conductive layers and/or measurement points or electrodes thereof, at least one other and preferably lower layer (e.g. lower when applied to a patient's skin) may not cover a section of said conductive layer(s). Differently put, not all layers may have to be arranged fully congruently with respect to one another. For example, an edge portion or end of one (preferably conductive) layer may not be covered by another (preferably lower and/or non-conductive) layer. Instead, it may project or protrude further to the sides of the layer structure and/or may have a larger footprint. Thus, its measurement points and in particular at least one electrode thereof may be directly opposite and/or connectable to the patient's skin.

In other words, according to this aspect, at least one conductive layer may have portion (e.g. a side or edge potion) that preferably comprises at least one or a plurality of electrode(s) and that is not obstructed and/or electrically isolated form the skin by an intermediated layer. Note that the respective non-obstructed section may be larger than the footprint of an electrode of said conductive layer, e.g. may be at least two or at least five times as large. It may define a larger non-isolated section than provided by the channel-like recesses discussed herein whose footprint is typically limited to that of the electrode and/or does not significantly deviate therefrom. Providing a respectively large non-covered section of a conductive layer may be an efficient way (e.g. with respect to manufacturing) for achieving reliable conductive connections between the skin and the conductive layer.

According to a further example, at least one conductive path of at least one of the conductive layers has at least one non-linear or non-straight section that is straightenable when stretching the layer structure. This may include a curved, bent, angled, zig-zag-shaped, rolled or other non-linear section being pulled and/or extended into a more linear shape, thus being straightened. Figuratively speaking, the nonlinear section may represent a reserve section or fallback section that may selectively assume a more linear shape to limit stresses (in particular tensile stresses) within the conductive path when stretching the layer structure.

Generally, the above embodiment enables an adjustable positioning of a conductive path and in particular electrode connected thereto. For doing so, the non-linear part acts as a dedicated deformable and in particular straightenable section.

The invention also relates to a method for producing a layer structure for application to a surface of a subject, in particular for use in medical products, the method comprising:
- providing a first conductive layer having at least one electrically conductive path; and
- providing at least a further conductive layer having at least one electrically conductive path;
- providing at least one intermediate layer extending at least partially between the first and further conductive layer;
wherein the first and further conductive layer are provided so that at least portions thereof are arranged above one another or, differently put, are stacked within the layer structure; and preferably wherein the layer structure is elastically stretchable.

Generally, the method may comprise any additional measures and features to produce a layer structure according to any of the embodiments disclosed herein. Also, any of the teachings, explanations, aspects and variants disclosed herein in connection with the layer structure may equally apply to and be valid for the method.

The method may include attaching the provided layers directly or indirectly (via at least one further layer extending therebetween) to one another. This may be done according to any of the following examples and generally by e.g. lamination, adhesion or ultrasonic welding.

According to a preferred embodiment, at least one of the first and further layer is printed on or attached to the intermediate layer. For example the intermediate layer may be provided and/or produced first to the then provided conductive layer at a surface thereof (e.g. at its top or bottom face). This may reduce the overall number of production steps as e.g. no dedicated base layers for printing the conductive paths of the conductive layer thereon need to be provided first. Instead, the printing may directly be done on the intermediate layer.

In one example, the first and further conductive layer are printed at or attached to opposite sides of the intermediate layer. This way, the intermediate layer may act as a printing substrate or printing base for both conductive layers. Again, this may limit the number of overall layers that need to be produced and thus the associated number of production steps, production costs as well as the resulting weight.

According to a further example, the intermediate the layer is printed on or attached to at least one of the first and further conductive layer (or to a layer to which the conductive layers are attached or on which they are deposited). For example, the intermediate layer may be laminated to a conductive layer (e.g. to a base layer thereof on which the conductive paths are printed).

Still further, the first conductive layer may be provided at a first substructure of the layer structure and the second conductive layer may be provided at a second substructure of the layer structure. The first and second substructure may be joined to form at least part of the layer structure. For example, the conductive layers may be printed on layers forming the substructures. These layers (and thus the substructures) may then be joined (e.g. laminated). The layers may have to be provided in layer structure anyway, e.g. to provide functions apart from acting as substructures. They may e.g. be intermediate and/or insulative layers or adhesive layers. This may enable a quick and reliable production, e.g. due to an underground for printing the conductive paths being directly available.

### Brief description of the drawings

Embodiments of the invention are described in the following with reference to the attached schematic drawings. Same features are marked with same reference signs throughout the figures.
- Fig. 1: is an explosive view of a layer structure according to an embodiment of the invention.
- Fig. 2: is a view of the layer structure with the single layers of Fig. 1 being joined.
- Fig. 3: is an explosive view of a layer structure according to a further embodiment of the invention.
- Fig. 4: illustrates a bending and/or flipping a layer structure according to an embodiment of the invention.

### Detailed description of the drawings

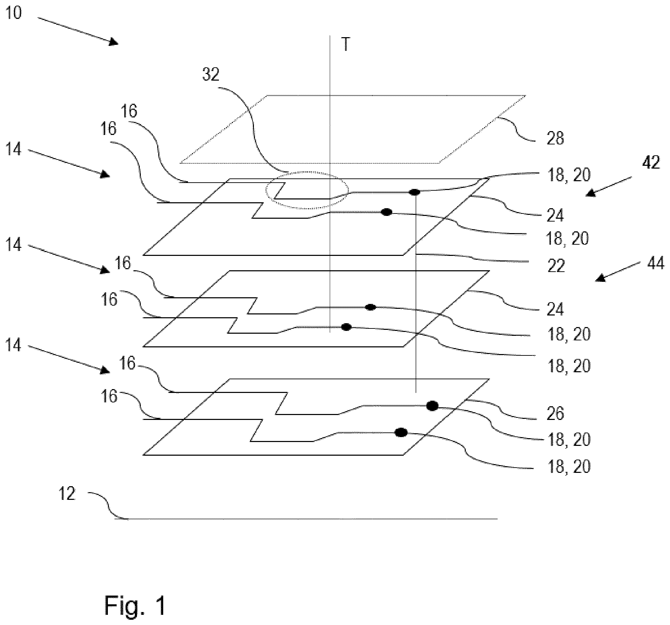

Figure 1 shows the single layers of a layer structure 10 according to an embodiment of the invention. The layer structure 10 is to be attached to a subject's surface 12, e.g. to the skin of a human patient. The exemplary layer structure 10 is an electrode patch that is e.g. usable for performing ECG.

As a mere example, the layer structure 10 comprises three conductive layers 14. Each conductive layer 14 consists of a deposition of conductive materials that form conductive paths 16. This deposition may e.g. be produced by screen printing. The area, plane or level of the layer structure 10 in which at least one and in the shown example two conductive paths 16 are provided forms a conductive layer 14. Alternatively and as indicated above, the conductive layers 14 may comprise base layers on which the conductive paths 16 are positioned.

Generally, each layer of the layer structure may extend substantially orthogonally to a thickness axis T which extends orthogonally to a main plane of the layer structure 10 and/or to the subject's surface 12.

Each conductive path 16 ends at a measurement point 18 that is part of an electrode 20. As discussed in detail above, the electrode 20 may comprise a recess 22 that extends through or across all layers between a measurement point 18 and the object's surface 12. Differently put, the recess 22 may extend through or across all layers below of the conductive layer 14 that comprises the respective measurement point 18.

A respective extension of the recess for (in Fig. 1) the uppermost measurement point 18 is indicated by a dotted line 22. It runs across the conductive layers 14 below of said measurement point 18 as well as through layers 24, 26 on which the conductive layers 14 are placed so that it reaches the subject's surface 12.

Two of these layers are intermediate layers 24 that are placed in between two conductive layers 14. For example, at least one conductive layer 14 may be printed on one (in Fig. 1 upper) surface of each intermediate layer 24. Further, each intermediate layer 24 may be laminated with its opposite (in Fig. 1 bottom) surface to a layer below of it. Thus, each intermediate layer 24 may be sandwiched between two other layers and in the depicted case between and in contact with two conductive layers 14.

As a mere example, the upper conductive layer 14 of Fig. 1 may be printed on the upper intermediate layer 24 to from a first substructure 42 of the layer structure 10. Further, the middle conductive layer 14 of Fig. 1 may be printed on the lower intermediate layer 24 to form a second substructure 44. These substructures 42, 44 may then be joined, e.g. by lamination.

The intermediate layers 24 are preferably insulative, thus electrically insulating the conductive layers 14 from one another. They generally space the conductive layers 14 apart from one another and preferably act as thermal insulators.

A (in Fig. 1) lowermost layer 24 of the layer structure 10 is an adhesive layer. At its external surface facing the subject's surface 12, an adhesive is provided for securing the layer structure 10 at the patient.

Fig. 1 also depicts an optional shielding layer 28 that forms an external surface of the layer structure 10 facing away from the subject's surface 12. The shielding layer 28 may provide any of the shielding effects discussed above, but in particular an electromagnetic shielding. For doing so, it may comprise a conductive material and preferably an insulating material that electrically insulates said conductive material from the conductive layer 14 below.

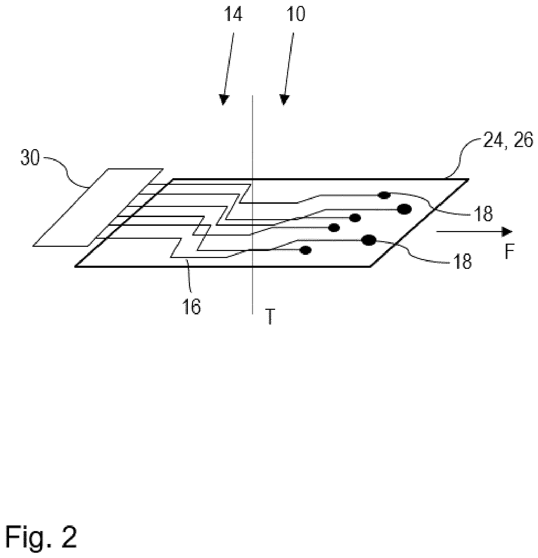

The layers 14, 24, 26, 28 are stacked on top of one another along the axis T. This results in the assembled layer structure 10 of Fig. 2. This means that the conductive layers 14 and in particular their conductive paths 16 are largely and preferably fully surrounded and thus shielded or encapsulated by adjacent layers 24, 26, 28. This provides protection from environmental impacts, such as radiation, dust and gases.

Preferably, each of the layers 24, 26 is optically transparent and/or radiolucent. Thus, when stacked, conductive paths 16 of the lower conductive layer 14 are still visible from outside as correspondingly indicated in Fig. 2. It is also evident that the conductive layers 14 are congruently arranged by being stacked or placed above one another within the layer structure 10. As a result, their conductive paths 16 may cross one another e.g. when viewed from above.

Further, each of the layers 24, 26 and preferably also the conductive paths 16 are elastically stretchable. At least the layers 24, 26 may largely or fully comprise a homogeneous polymeric material, such as TPU.

As a preferred option, a connector 30 is depicted. Said connector 30 is connected to each of the conductive paths 16 to receive electric signals therefrom. These signals stem from the electrodes 20 to which each conductive path 16 is connected. They thus correspond to electric signals at the subject's surface 12 that are picked up (i.e. captured) by the electrodes 20.

The connector 30 comprises an interface for connecting to an external device (not depicted). Said external device comprises further electrical components for analyzing the electrical signals. This means that the layer structure 10 is electrically passive which makes it economically disposable. The external device, on the other hand, may have longer lifespans and may e.g. be used on different patients. It may selectively be connected to disposable layer structures 10 that are applied to each specific patient.

In consequence, the disclosed layer structure 10 may generally represent an electrically passive signal bus and/or passive signal transfer device by means of which electric signals may be (passively) picked up and (passively) conducted to the connector 30.

In the depicted embodiment, the conductive paths 16 do not have a linear and/or straight course. Instead, they have at least one angled portion 32 (see Fig. 1) that could also be curled, bent or have a zig-zag-shape. This portion 32 supports a deformation of the conductive paths 16 when trying to manually adjust positions of the electrodes 20 by stretching the layer structure 10. Specifically, it allows for the conductive paths 16 to assume a more straight and less angled shape while limiting local stresses within the paths 16 when a pulling force F is applied by a practitioner to the layer structure 10.

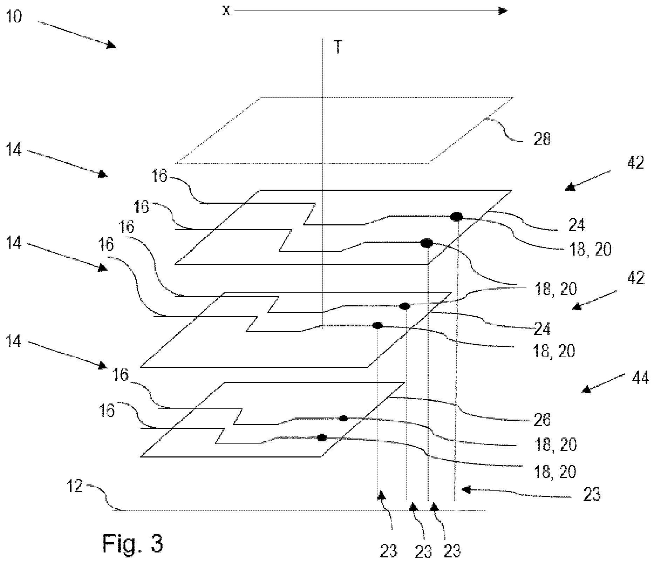

Fig. 3 illustrates a further embodiment which is based on the embodiments of Figs. 1-2 so that the same reference signs are used. A difference to said previous embodiment of Figs. 1-2 is the conductive connection of the electrodes 20 of at least some conductive layers 14 to the skin 12. Yet, as detailed below, the ways of providing said conductive connections according to Figs. 1-2 and according to Fig. 3 may also be combined, i.e. may jointly be provided within one layer structure 10.

In Fig. 3, instead of forming such a connection with help of preferably gel-filled recess 22 depicted in Fig.1, at least some of the electrodes 20 of some conductive paths 16 may be exposed, i.e. may not be covered by some other layer, in particular not by a non-conductive layer extending in between said conductive path 16 and/or electrode 20 and the skin 12. Put differently, at least some of the electrodes 20 of some conductive paths 16 are not electrically isolated or obstructed from forming a conductive contact with the skin 12 by any further layers.

In Fig. 3, this is achieved by adjusting an extension of at least one intermediate layer 24 below of at least one conductive layer 16, with "below" indicating a position closer to skin 12. As a mere example, in Fig. 3 an x-axis indicates an extension orthogonally to the thickness axis T and thus within or in parallel to a main plane of the layer structure 10. In Fig. 3, the extension of the layers 24, 26 differs and, viewed along the thickness axis T and towards the skin 12, decreases along the x-axis from layer to layer 24, 26. This means that the uppermost intermediate 24 layer forms an (with respect to the x-axis) outermost edge of at least the depicted section of the layer structure 10. The adjacent and lower intermediated layer 24 is recessed relative to said upper intermediated layer 24 along the x-axis, whereas the lowermost adhesive layer 26 is recessed even further. In place of the recessed extension, cut-outs could be provided in the respective layers 24, 26 that define similar non-covered portions of the respective layers 24, 26 above.

As indicated by dotted lines 23, the electrodes 20 of the intermediated layers 24 are thus unobstructed with respect to and/or from the skin 12 and can directly (or with help of a non-depicted conductive gel applied to the skin 12) conductively contact the skin 12. In particular in edge regions of the layer structure 10, this may represent a suitable alternative and a possibly cheaper way of enabling conductive connections between the skin 12 and electrodes 20 compared to providing dedicated channel-like recess 22 as in Fig. 1.

Note that the non-obstruction of electrodes 20 or of general electric contacts of a conductive layer 14 may also be provided by cut-outs within non-conductive layers arranged below, said cut-outs preferably having larger sizes compared to single-electrode recesses and e.g. covering a number of electrodes 20.

Of course, it also possible to combine the embodiments of Figs. 1-3, so that for some electrodes 20 recess 22 according to Fig. 1 are provided, whereas some electrodes 20 remain unobstructed and/or exposed according to Fig. 3.

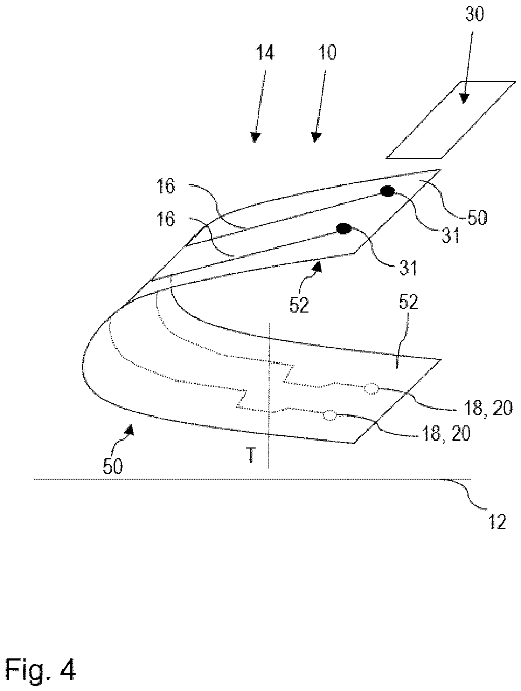

Fig. 4 illustrates an optional aspect of bending and/or flipping a conductive layer structure 10 of any of the kinds disclosed herein in order to connect it to a connector 30. Note that this aspect is not limited to multi-layer structures 10 disclosed herein (i.e., "multi" indicating a plurality of conductive layers 14), but can also be implemented in the context of such multi-layer structures 10 as exemplified throughout Figs. 1-3. Therefore, the same references as in Figures 1-3 are used. However, the aspect of Figure 4 can also be used for layer structures 10 having only one conductive layer 14, but apart from that may comprise any further features disclosed herein.

Fig. 4 again indicates a patient's skin 12 to which the layer structure 10 is to be applied. The layer structure 10 faces the skin 12 with its (in Fig. 4) underside 50. At said underside 50 an adhesive layer 26 similar to Fig. 1 may be provided. On its upperside 52 facing away from the patient's skin 12, any preferably non-conductive material and/or non-conductive layer may be provided, e.g. a shielding layer 28 similar to Fig. 1.

As a mere example, only on single conductive layer 14 is provided. Its conductive paths 16 are preferably placed closer to the underside 50 than to the upperside 52. They may form part of and/or partially extend within the underside 50. The conductive paths 16 are preferably separated from said upperside 52 by a (preferably non-conductive) material along their complete length. The conductive paths 16 again comprise and/or end at electrodes 20 for measuring biosignals according to any of the aspects disclosed herein.

For the reasons set out below, the layer structure 10 of Fig. 4 is foldable so as to assume a C-shape. This means that part of the underside 50, as a result of the folding, does not face the patient's skin 12 any longer but in fact faces away therefrom. Similarly, part of the upperside 50 as a result of said bending faces towards the patient's skin 12. In fig. 4, these respectively re-oriented parts of the layer structure 10 are comprised by an initially (i.e. before bending) left end section of the layer structure 10. The re-orientation may also be referred to as flipping said portion of the layer structure 10, e.g. so that the initial underside 50 forms the upperside 52 and vice versa.

The positioning of the conductive paths 16 closer to and/or within the underside 50 is illustrated in figure 4 by using either dotted or continuous lines. Specifically, when viewed and/or oriented so as to be covered by the upperside 52, the conductive paths 16 are to a larger extent shielded by a preferably non-conductive material. In such a state or at such an orientation, the conductive paths 16 and electrodes 20 are depicted with dotted lines. On the other hand, when viewed and/or oriented so as form the underside 50, the conductive paths 16 are to a lesser extent shielded by a preferably non-conductive material or are exposed in and/or form part of said underside 50. In such a state or at such an orientation, the conductive paths 16 are depicted with continuous lines.

In consequence, in Fig. 4 the conductive paths 16 and electrodes 20 extending in the non-bent section of the layer structure 10 that faces and extends along the skin 12 are depicted with dotted lines. The conductive paths 16 extending in the bent or flipped section of the layer structure 10 are depicted with continuous lines as they face the viewer.

With this configuration, the conductive paths 16 in the flipped section are more easily accessible for connecting a connector 30 thereto. For example, the connector 30 can be easily placed on top of said conductive paths 16 and in particular on optional connecting sections 31 thereof which would not be possible in a non-bent configuration. The only schematically indicated connecting sections 31 can be configured as open ends or traces of a the conductive paths 16 and can generally provide a connection interface for conductively connecting to the connector 30.

Note that the flipping and the thereby improved accessibility also reduces requirements for structurally adapting a section of the layer structure 10 to which the connector 30 should connect. For example, no part of the upperside 52 has to be locally removed to gain access to any conductive paths 16 of a non-flipped layer structure 10.

## Claims

1. Conductive Layer structure (10) for application to a surface (12) of a subject, in particular for use in medical products, comprising:
• a first conductive layer (14) having at least one electrically conductive path (16);
• at least a further conductive layer (14) having at least one electrically conductive path (16);
• at least one intermediate layer (24) extending at least partially between the first and further conductive layer (14);
wherein at least portions of the first and further conductive layer (14) are arranged above one another within the layer structure (10);
and wherein the layer structure (10) is elastically stretchable.

2. Layer structure (10) according to claim 1, **characterised in that** the intermediate layer (24) is an insulative layer and/or **in that** the first and further conductive layer (14) as well as the intermediate layer (24) are non-movable relative to one another and/or are at least indirectly secured to one another.

3. Layer structure (10) according to claim 1 or 2, **characterised by** comprising a layer that is configured to provide a shielding effect for at least one of the first and further conductive layer (14), the shielding effect relating to limiting at least one of the following:
• a forming of electrically conductive connections;
• an electrostatic coupling;
• an electromagnetic induction;
• a radio frequency interference;
• environmental impacts, such as humidity.

4. Layer structure (10) according to claim 3, **characterised in that** the layer providing the shielding effect is formed by one of the first and further conductive layer (14) and provides a shielding effect for the respective other of the first and further conductive layer (14).

5. Layer structure (10) according to any of the previous claims, **characterised in that** at least one of the first and further conductive layer (14) is free of active electric components or comprises more passive electric components than active electric components.

6. Layer structure (10) according to any one of the preceding claims, **characterised in that** the first and further conductive layer (14) are conductively connected to one another and/or that the first and further conductive layer (14) are conductively connected or connectable to a common component in form of a connector (30) that is conductively connected or connectable to an internal or external device so that signals can be transferred from the conductive paths (14) to the device via the connector (30).

7. Layer structure (10) according to any one of the preceding claims, **characterised in that** at least one layer of the layer structure (10) comprises or is attached to a thermoplastic polymer material, in particular a thermoplastic polyurethan material.

8. Layer structure (10) according to any of the previous claims, **characterised in that** at least one of the first and further conductive layer (14) has a conductive path (16) that is electrically connected to and/or is part of a measurement point (18) for capturing electric signals at the subject's surface (12).

9. Layer structure (10) according to claim 8, **characterised in that** a recess (22) is provided which extends from the measurement point (18) across all layers (14, 24, 26) between the measurement point (18) and the subject's surface (12).

10. Layer structure (10) according to any of the previous claims,
**characterised in that** at least one conductive path (16) of at least one of the conductive layers (14) has at least one non-straight section (32) that is straightenable when stretching the layer structure (10).

11. Method for producing a conductive layer structure (10) for application to a surface (12) of a subject, in particular for use in medical products, the method comprising:
• providing a first conductive layer (14) having at least one electrically conductive path (16); and
• providing at least a further conductive layer (14) having at least one electrically conductive path (16);
• providing at least on intermediate layer (24) extending at least partially between the first and further conductive layer (14);
wherein the first and further conductive layer (14) are provided so that at least portions thereof are arranged above one another within the layer structure (10); and
wherein the layer structure (10) is elastically stretchable.

12. Method according to claim 11, **characterised in that** at least one of the first and further conductive layer (14) is printed on or attached to the intermediate layer (24).

13. Method according to claim 11, **characterised in that** the first and further conductive layer (14) are printed at or attached to opposite sides of the intermediate layer (24).

14. Method according to claim 11, **characterised in that** the intermediate layer (24) is printed on or attached to at least one of the first and further conductive layer (14).

15. Method according to claim 11, **characterised in that** the first conductive layer (14) is provided at a first substructure (42) of the layer structure (10) and the further conductive layer (14) is provided at a second substructure (44) of the layer structure (10) and **in that** the first and second substructure (42, 44) are joined to form at least part of the layer structure (10).
